# EUROPEAN PATENT APPLICATION

(11) **EP 4 393 517 A1**
(43) Date of publication of application: **03.07.2024**
(21) Application number: 22866808.3
(22) Date of filing: 26.09.2022
(51) Int. Cl.: A61K 49/22, A61K 31/00, A61K 33/00

(54) **METAL COMPOSITE FOR ULTRASONIC CONTRAST AND USE THEREOF**

(30) Priority: 09.09.2021 US 202163242045 P
(71) Applicant: Original Biomedicals Co., Ltd., Tainan City 744 (TW)
(72) Inventor: CHEN, Jia-long, Tainan, Taiwan 744 (CN); LIAO, Wei-chuan, Tainan, Taiwan 744 (CN); SUN, Tzu-hui, Tainan, Taiwan 744 (CN); CHEN, Chia-hung, Tainan, Taiwan 744 (CN); WANG, Chau-hui, Tainan, Taiwan 744 (CN); YEN, Hsiao-pao, Tainan, Taiwan 744 (CN)
(74) Representative: Cabinet Chaillot
(86) International application number: PCT/CN2022/121437
(87) International publication number: WO 2023/036347

(57) **Abstract**

A metal composite for ultrasonic contrast. The metal composite has a metal nano-structure and comprises a contrast core and a shell ligand coated on the contrast core. The contrast core includes metal ions, and the shell ligand includes a polymer having a chelating group, wherein the chelating group is a Lewis base which forms a coordination covalent bond with the metal ions to form a stable metal nano-structure; and the metal ions can effectively generate echoes of ultrasonic waves, enhance the brightness and contrast of the image, and help stabilize the metal nano-structure in the form of Lewis acid-base pair chelation, so that the metal ions will not easily disintegrate during circulation in the human body, thereby meeting the needs of long-time contrast imaging..

## Description

### FIELD OF THIS INVENTION

This disclosure is related to a metal composite, and especially a metal composite having a main structure formed of Lewis acid-base pairs; this disclosure is also directed to a use of the metal composite for ultrasonic contrast of organs or tissues of a living organism.

### BACKGROUND OF THIS INVENTION

The commercial ultrasonic contrast agents are micron-scale microbubbles, such as SonoVue, Definity, Luminity, Sonazoid. The commercial metal composite is essentially consisted of insoluble gas (perfluorocarbon or sulfur hexafluoride) and encapsulating shell (lipids, proteins or polymers). In the past few decades, these metal composites have been applied to organ or tissue imaging clinically, and cavitated microbubble structure is growing significant in ultrasound diagnosis and therapeutic applications. In addition, microbubbles acting as carriers or enhancer of drugs or genetic materials have also drawn wide attentions.

Nevertheless, the current commercial microbubbles are featured of large sizes and shorter half-life, and both conditions render their diffusion into target tissue less efficiently. Endothelial pores within a tumor tissue, for example, are normally sized of 400 to 800 nm, which limits targeting function of the commercial microbubbles owing to their large sizes. The commercial microbubbles are also prone to collapse under ultrasound, so the their residence time in circulation of a human body is short (short half-life). Moreover, cavitation upon burst of the microbubbles may induce tissue inflammation as well. In one more aspect, when the microbubbles are excessively concentrated, hemostasis may be induced, or risk of thrombosis may rise due to outer shells of the microbubbles. As a result, upper limit of the dose inevitably undermines microbubble application in long term ultrasound diagnosis or surveillance.

### SUMMARY OF THIS INVENTION

To overcome technical limitations accounted by sizes and circulatory residence time of the aforementioned microbubble ultrasonic contrast agents, this invention provides a nano-micelle metal composite, based on a metal nano-structure, which is characterized by small size and lengthy circulatory residence time. The metal nano-structure takes a Lewis acid as the core to attract lone pairs from a Lewis base ligand, and forms a stable metal nano-structure in a chelating manner. The metal nano-structure can also be applied as a nano-scale ultrasonic contrast contrast agent that alters ultrasound absorption, reflection and conduction. Furthermore, metallic drugs are primarily applied to computed tomography (CY) or magnetic resonance imaging (MRI), while the nano-micelles having metal ions able to alter ultrasound absorption and conduction coefficients in a living organism, such as reflection, refraction and diffraction. In light of this, the metal nano-structure has great potential for a use to enhance ultrasonic contrast imaging.

The present invention provides a metal composite for ultrasonic contrast, and the metal composite comprises a contrast core comprising a metal ion; and a shell ligand comprising a polymer having a chelating group, wherein the chelating group is a Lewis base and forms a coordinate-covalent bond with the metal ion so as to form a stable metal nano-structure.

Preferably, the contrast core comprises an contrast ligand, and the contrast ligand has a Lewis base functional group, and forms coordinate-covalent bond with the metal ion via the Lewis base functional group, wherein the Lewis base functional group of the contrast ligand comprises any one of a primary carbon having a π bond, a secondary carbon, a group 16 element, a primary amine, a secondary amine, a tertiary amine or a combination of two or more thereof.

Preferably, the contrast ligand comprises a chemical structure as a constitutional formula (1): 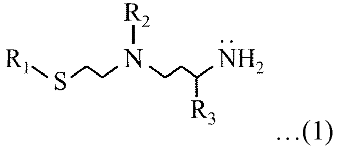 wherein R₁ is selected from a group consisting of hydrogen, hydroxyl group, carboxylic group, alcoholic group, ketone group, furans, amine, anilines, pyrroles, esters, amides, imines, pyridines, pyrimidins, imidazoles, pyrazols, phosphonic acids, sulfide and disulfide; R₂ is selected from a group consisting of hydrogen _{'} hydroxyl group, carboxylic group, alcoholic group, ketones, furans, amines, anilines, pyrroles, thiols, esters, amides, imines, pyridines, pyrimidines, imidazoles, pyrazols, sufonamides and phosphonic acid; R₃ is selected from a group consisting of hydrogen, hydroxyl group and carboxylic group.

Preferably, the metal ion is selected from a group consisting of Fe, Cu, Ni, In, Ca, Co, Cr, Gd, Al, Sn, Zn, W, Sc, Ti, Mn, V, Mg, Be, La, Au, Ag, Cd, Hg, Pd, Re, Tc, Cs, Ra, Ir, Ga and a combination of two or more thereof.

Preferably, the chelating group is selected from a group consisting of poly(aspartic acid), poly(glutamic acid), polylysine, poly(acrylic acid), polyethyleneimine, poly(methacrylic acid), hyaluronic acid, poly(N-isopropyl acrylamide), poly(lactic acid), poly(butylenesuccinate) and a combination of two or more thereof.

Preferably, the polymer further comprises a dispersing group selected from a group consisting of poly(ethylene glycol), chitosan, collagen, poly(N-isopropyl acrylamide), amylose, cellulose, poly(hydroxybutyrate), poly(caprolactone), carboxymethylcellulose, dextran, cyclodextrin and a combination of two or more thereof.

In another aspect, the present invention further provides a use of a metal composite for preparation of an organism imaging composition, comprising administrating an effective dose of the organism imaging composition to a vasculature of a location in need, wherein the organism imaging composition comprises the aforementioned metal composite.

The metal composite provided in the present invention demonstrates the following advantages, and being beneficial for clinical medical imaging application:

Firstly, unlike conventional bubble contrasts that tend to burst under ultrasound, the metal composite is small sized and structurally stable. The metal composite shows more longer circulatory residence time within a biological fluid, which satisfies demands of long term imaging with contrast enhancement in a specific organ or tissue. The nano-scale size of the metal composite not only allows its entry into tissue interiority of a specific organ, but also provides sterilization convenience, which lowers risk of microbial infection when using the contrast.

Secondly, the metal composite is applicable for a variety of organs or tissues including heart valves, pericardium, liver or tumor tissues where blood circulation could arrive. The metal composite presents excellent reflection of ultrasound echo and higher level of contrast enhancement so that the contrast imaging demonstrates brightness and resolution at high level, and its lengthy residence time assists long-term high-resolution observations.

### BRIEF DESCRIPTION OF DRAWINGS

Fig. 1 illustrates a method for making the metal composite in this invention;
Figs. 2A and 2B show the structures of Amifostine and its derivatives;
Fig. 3 shows the structure formula of PEG-b-PGA;
Fig. 4 shows the metal composite and its structure formula;
Fig. 5 shows gel chromatography analysis of PEG-b-PGA;
Fig. 6 shows Proton Nuclear Magnetic Resonance (¹H-NMR) spectrum of PEG-b-PGA;
Fig. 7 shows particle size analysis of the metal composite;
Figs. 8A and 8B demonstrate cardiac ultrasound images of rat hearts at systolic and diastolic phases in experimental example 1;
Figs. 9A to 9B demonstrate cardiac ultrasound images of rat hearts at systolic and diastolic phases in experimental example 2;
Fig. 9C demonstrates a continuous cardiac ultrasound images with the metal composite in this invention;
Figs. 9D to 9E illustrate brightness and intensity analysis of the cardiac ultrasound images in experimental example 2;
Fig. 10A illustrates cardiac ultrasound images of rat hearts at systolic and diastolic phases in experimental example 3;
Figs. 10B to 10C illustrate brightness and intensity analysis of heart valve images;
Fig. 10D illustrates the sampling region of pericardium images for data analysis;
Figs. 10E to 10F illustrate brightness and intensity analysis of outer pericardium images in experimental example 3; and
Fig. 11 illustrates images of rat hepatic ultrasound images in experimental example 4.

### DETAILED DESCRIPTION OF THIS INVENTION

The content of this invention is concretely illustrated through several embodiments, but these are not intended to limit the scope of the patent application of this invention. It should be understood that the invention can also be modified in many ways, and thus should be interpreted within the scope of the appended claims. Apart from the details described in these embodiments, the invention can also be broadly implemented in other embodiments. Any equivalent alterations or modifications accomplished without deviating from the spirit disclosed by this invention should be included within the scope of the patent application described below

In one aspect, the present invention provides a metal composite for ultrasonic contrast, and the metal composite comprises a contrast core comprising a metal ion; and a shell ligand comprising a polymer having a chelating group, wherein the chelating group is a Lewis base and forms a coordinate covalent bond with the metal ion so as to form a stable metal nano-structure. A coordinate covalent bond can also be regarded as one type of covalent bonds. When the shared electron pair in the covalent bond is provided solely by one atom, the covalent bond is regarded as a coordinate covalent bond. Formation of the coordinate covalent bond requires a central atom or ion having vacant orbitals to accept the lone pair of electrons from the ligand. For example, Lewis acid-base pair forms a compound through coordinate-covalent bond. Comprehensibly, Lewis base provides a lone pair to Lewis acid so as to form a coordinate covalent bond and results in a coordinate compound. In the coordinate compounds, the atom having larger electronegativity accepts the lone pair provided by the atom having smaller electronegativity, wherein the former receives a lone pair and presenting negative valence, while the latter gives away a lone pair and presenting positive valence. The coordinate covalent bond is a covalent bond with high stability, which renders the metal nano-structure have less tendency of disassembly and demonstrate longer residence during imaging.

Preferably, the metal ion to form the contrast core is selected from a group consisting of Fe, Cu, Ni, In, Ca, Co, Cr, Gd, Al, Sn, Zn, W, Sc, Ti, Mn, V, Mg, Be, La, Au, Ag, Cd, Hg, Pd, Re, Tc, Cs, Ra, Ir, Ga and a combination of two or more thereof.

In some preferred embodiments, the contrast core comprises an contrast ligand, and the contrast ligand has a Lewis base functional group, and forms coordinate-covalent bond with the metal ion via the Lewis base functional group, wherein the Lewis base functional group of the contrast ligand comprises any one of a primary carbon having a π bond, a secondary carbon, a group 16 element, a primary amine, a secondary amine, a tertiary amine or a combination of two or more thereof. It is understood that these functional groups have high electronegativity or a lone pair of electrons in the orbital thereof to provide for the metal ion, and to form a coordinate-covalent bond to stabilize the contrast core. Specifically, the contrast ligand comprises but not limited to one Lewis base functional group, there can be one or more Lewis base functional groups on the Lewis base functional group. Moreover, in addition to the aforesaid nitrogen-containing functional groups, in some embodiments, the aforesaid Lewis base functional groups can be functional groups containing atoms with high electronegativity such as sulfur-containing functional groups or phosphor-containing functional group.

Preferably, the contrast ligand comprises a chemical structure as a constitutional formula (1): 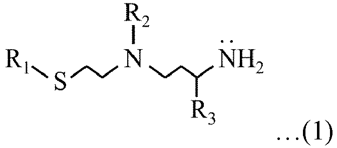 wherein R₁ is selected from a group consisting of hydrogen, hydroxyl group, carboxylic group, alcoholic group, ketone group, furans, amine, anilines, pyrroles, esters, amides, imines, pyridines, pyrimidins, imidazoles, pyrazols, phosphonic acids, sulfide and disulfide; R₂ is selected from a group consisting of hydrogen _{'} hydroxyl group, carboxylic group, alcoholic group, ketones, furans, amines, anilines, pyrroles, thiols, esters, amides, imines, pyridines, pyrimidines, imidazoles, pyrazols, sufonamides and phosphonic acid; R₃ is selected from a group consisting of hydrogen, hydroxyl group and carboxylic group. specifically, when R₃ is a carboxylic group, the contrast ligand is an amino-acid derivative having both hydrophilicity and Lewis base functional group. the amino-acid derivative can not only form a coordinate-covalent bond with the contrast core, but also, with the amino-acid structure, enhance biocompatibility in the biofluids, which benefits carcinoma-targeting vector design.

In some preferred embodiments, the contrast ligand can be exemplified by Amifostine _{'} Amifostine thiol (WR-1065), Amifostine disulfide (WR-33278), Doxorubicin, Docetaxel, Epirubicin, Amphotericin, Ciprofloxacin or a combination of two or more thereof, or derivatives thereof. For instance, the contrast ligand can be exemplified by Amifostine having five portions to provide lone pairs, and these portions are located on two -OH, -P=O, -NH, and -NH₂, respectively. The contrast ligand can also be exemplified by Amifostine thiol (WR-1065) having three portions to provide lone pairs, and these portions are located on -SH, -NH, and -NH₂, respectively. the contrast ligand can be further exemplified by Amifostine disulfide (WR-33278) having 6 portions to provide lone pairs, and these portions are located on -SH, -NH, and -NH₂, respectively. The aforementioned contrast ligands have multiple sites to provide lone pairs to chelate the metal ion so that metal nano-structure can be stabilized.

In the aforementioned embodiments, the polymer can be formed by multiple chelating groups and multiple dispersing groups. The polymer is a Lewis base to form a coordinate covalent bond with the metal ion when covering thereon. The polymer can be exemplified by unidentate ligands, bidentate ligands, tridentate ligands, hexadentate ligands, or polydentate ligands, and the molecular weight is 1,000 to 100,000 Daltons. The polymer can be formed by intercalation and copolymerization of the chelating groups and the dispersing groups, and can also be referred to as a block copolymer.

In some preferred embodiments, the chelating group, as being the Lewis base functional group, can be exemplified by poly(aspartic acid), poly(glutamic acid), polylysine, poly(acrylic acid), polyethyleneimine, poly(methacrylic acid), hyaluronic acid, poly(N-isopropyl acrylamide), poly(lactic acid), poly(butylenesuccinate) or a combination of two or more thereof.

To ensure the metal composite stability and well-dispersed in biofluids, the dispersing group can be chosen from polyethylene glycol), chitosan, collagen, poly(N-isopropyl acrylamide), amylose, cellulose, poly(hydroxybutyrate), poly(caprolactone), carboxymethylcellulose, dextran, cyclodextrin or a combination of two or more thereof.

In sum, when the block copolymer covers on the contrast core to form the metal nano-structure, the dispersing group without chelating competence extends from the outside of metal nano-structure. Through interaction of hydrophilic functional group provided by the block copolymer with a dispersing solvent or body fluid of an organism, dispersibility of the metal composite is enhanced. Moreover, the polymer contains multiple chelating groups, which renders the polymer possess many dentate ligands. These ligands and metal ions form multiple coordinate-covalent bonds, which allows the polymer, the contrast ligand and the metal ions to form a stable metal nano-structure. Long-term residence of the metal nano-structure in an organism can therefore be realized, and the metal nano-structure is biodegradable material with good dispersity, suitable for clinical application including long period imaging on organs or tissues, or delayed drug release.

In addition to applying the metal composite to ultrasonic contrast, the metal ion can also be used to realize applications including MRI magnetic resonance imaging, single photon emission computed tomography, positron emission tomography, internal radiation therapy, or leukocyte imaging. For example, Gd can be chosen for MRI magnetic resonance imaging; ^{99m}Tc can be chosen for single photon emission computed tomography; ⁶⁸Ga can be chosen for positron emission tomography; ¹⁸⁸Re can be chosen for internal radiation therapy; ¹¹¹In can be chosen for leukocyte imaging. Any applications of the metal nano-structure constructed through the above-mentioned coordinate-covalent bond principle in this invention are within the scope of protection of this invention.

In some exemplary embodiments, the metal composite comprises a contrast core comprising a metal ion and an contrast ligand, wherein the contrast ligand contains a Lewis base functional group to form a coordinate covalent bond with the metal ion; and a shell ligand comprising a polymer having a chelating group, wherein the chelating group is a Lewis base and forms a coordinate covalent bond with the metal ion so as to form a stable metal nano-structure, wherein the metal ion is selected from a group consisting of ferrous ion, ferric ion and tetravalent gallium ion, and the contrast ligand is Amifostine, Amifostine thiol (WR-1065), or Amifostine disulfide (WR-33278), and the polymer is polyethylene glycol)-b-poly(glutamic acid), wherein the chelating group is poly(glutamic acid) and the dispersing group is poly(ethylene glycol). The aforesaid polymer bonds with the ferrous ion, ferric ion or gallium ion through the chelating group, and disperses the metal composite in a stable form among the biofluids through the dispersing group, which is beneficial for the metal composite to arrive target location of imaging by circulation system or local diffusion. More specifically, the block copolymer, as the aforesaid polyethylene glycol)-b-poly(glutamic acid), has the highly biocompatible poly(glutamic acid) as being the chelating group (carboxylic group, -COO⁻) to form coordinate covalent bonds with ferrous ion, ferric ion or gallium ion. Meanwhile, the block copolymer has the highly hydrophilic poly(ethylene glycol) as being the dispersing group, which allows good dispersion of the metal composite in a stable form among the biofluid. To be explicit, in order to achieve the aforesaid good dispersibility, the polyethylene glycol) must by modified to have ligand functional groups, and such ligand groups are originally absent on the polyethylene glycol). The ligand groups are the so-called chelating group in this invention, and enable the polymer to for coordinate covalent bonds with the metal ions so as to link the ferrous ion to the poly(ethylene glycol) having good dispersibility. Moreover, the poly(glutamic acid) is a natural polymer that is bio-degradable, and ensures the biocompatibility of the metal composite provided in this invention.

To achieve the aforesaid purposes of this invention, in another aspect, the present invention provides a method for making a metal composite, comprising steps of:
Step S1: providing a raw material comprising Amifostine, PEG-b-PGA and FeCl₂;
Step S2: adding the material into a compatible buffer solution and proceeding a homogenous agitation procedure so that the Amifostine chelates the ferrous ion (Fe²⁺) to form a contrast core; furtherly, during the homogenous agitation procedure, the PEG-b-PGA chelates on the surface of the contrast core through chelating groups thereof, and finishing a self-assembly reaction to obtain a metal composite.

In some embodiments, the raw material contains 0. 1 to 10 mg Amifostine, 0. 1 to 100 mg PEG-b-PGA and 0.01 to 50 mg FeCl₂; more specifically, concentration of the Amifostine ranges from 0.0.1 to 10 mg/mL, the buffer solution is HEPES[4-(2-hydroxyethyl)-1-piperazinee-thanesulfonic acid)] at pH from 6.5 to 7.5, wherein the homogenous agitation procedure continues under 4 to 40°C until the self-assembly reaction is completed.

In one another aspect, the present invention further provides a use of a metal composite for making a bio-imaging composition, comprising administrating an effective dose of the bio-imaging composition to vasculature of a location in need, wherein the bio-imaging composition comprises the aforementioned metal composite.

In various embodiments, the location in need can be an organ or an tissue of any organism, such as mediastinum tissue or abdominal tissue of a human body, but not limited to this. In some particular embodiments, the bio-imaging composition can arrive at human organ tissue such as heart, liver, spleen, lung, kidney, pancreas, esophagus, stomach, intestine, colon, lymphatic system or reproductive system so that ultrasonic contrast at these locations can be enhanced. In other embodiments, the bio-imaging can be used for tumor tissue imaging in order to label the contour of a tumor tissue or to penetrate further inside the tumor tissue.

In the aforesaid use, the bio-imaging composition can be manufactured by mixing a pharmaceutically acceptable carrier and the metal composite, and further being administrated to a location in need. Primarily, the route of administration is intravenous injection, but not limited to this. The "effective dose" in this invention refers to a weight ratio of the metal composite to the subject, and can be present with a unit of mg/kg. In some embodiments, 8 week-old SD rats are administrated of the bio-imaging composition containing the metal composite at a dose from 1 to 100 mg/kg. For conversion of the effective dose into human equivalent dose (HED) according to U.S. Food and Drug Administration (FDA), taking a human adult for example, the conversed administration dose is 0.1 to 16.0 mg/kg, and preferably from 0.80 to 6.50 mg/kg.

The focus of this invention discussed here is on ultrasonic contrast functionality. To thoroughly understand this invention, detailed structures, their components, and method steps will be presented in the following description. It is apparent that the implementation of this invention is not limited to the specific details familiar to those skilled in the art of ultrasonic contrast. On the other hand, well-known structures and their components are not described in detail to avoid unnecessarily limiting this invention. Furthermore, to provide a clearer description and enable those skilled in the art to understand the content of this invention, the parts in the illustrations are not drawn to scale. Some dimensions are exaggerated in relation to other relevant scales for emphasis, and irrelevant detailed parts are not fully depicted for simplicity in the illustrations. The preferred embodiments of this invention will be described in detail below. However, apart from these detailed descriptions, this invention can also be broadly implemented in other embodiments, and the scope of this invention is not limited to these embodiments but is defined by the subsequent patent claims.

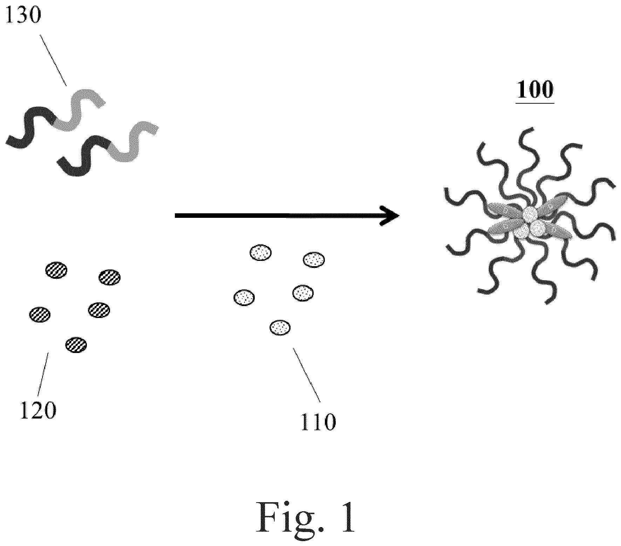

Accordingly, examples are provided hereinafter to illustrate manufacturing, specific structure, and stability test of the metal composite present in this invention. As shown in Fig. 1, the method for making the metal composite (100) comprises: preparing 0.1 to 10 mg cell protectant Amifostine (110), 0.1 to 100 mg PEG-b-PGA (120) and 0.01 to 50 mg FeCl₂(130); then adding the mixed raw material in 1 to 10 mL buffer solution, and proceeding a homogenous agitation under 4 to 40°C for 0 to 48 hours so as to obtain metal composite (100) of particle size at 20 to 50 nanometers, and the buffer solution is 0.05M HEPES(4-(2-hydroxyethyl)-1-piperazineethane-sulfonic acid) at pH 6.5 to 7.5.

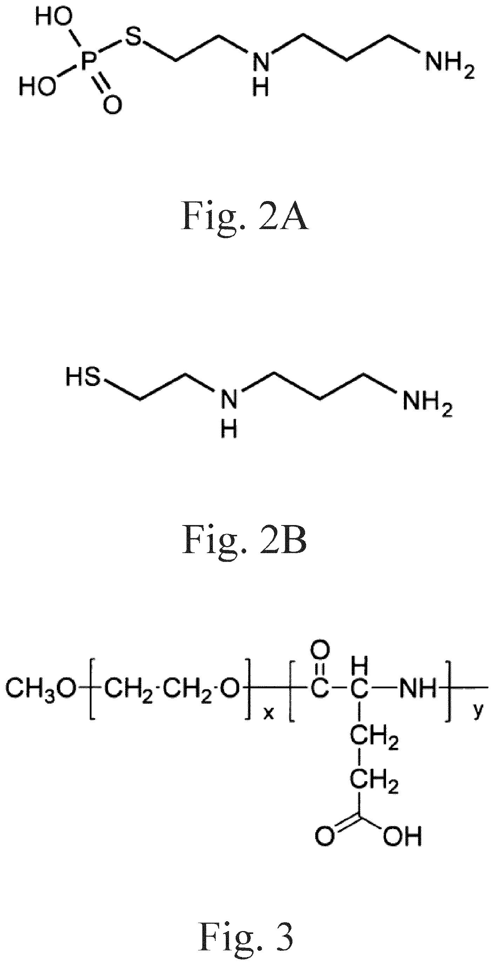

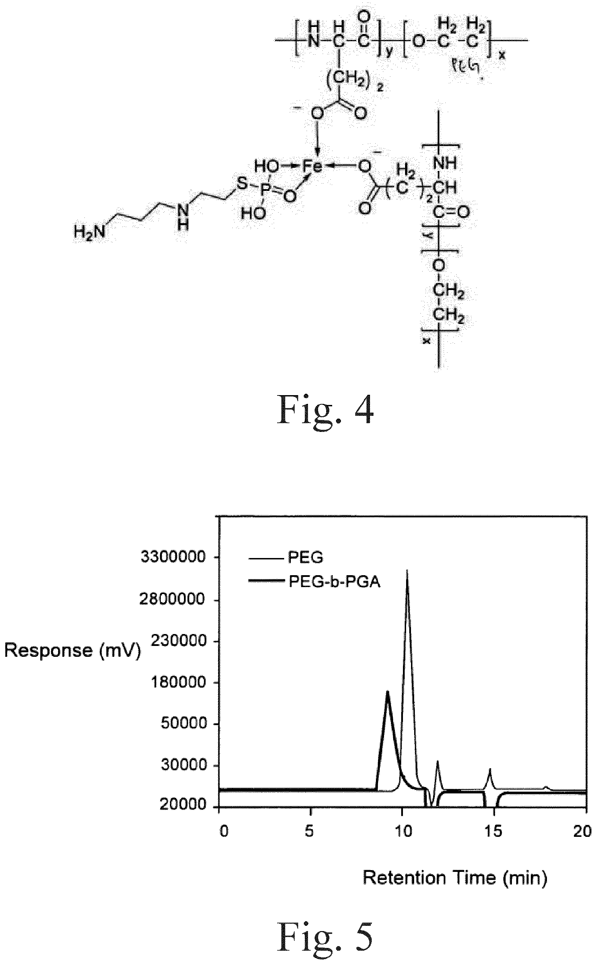

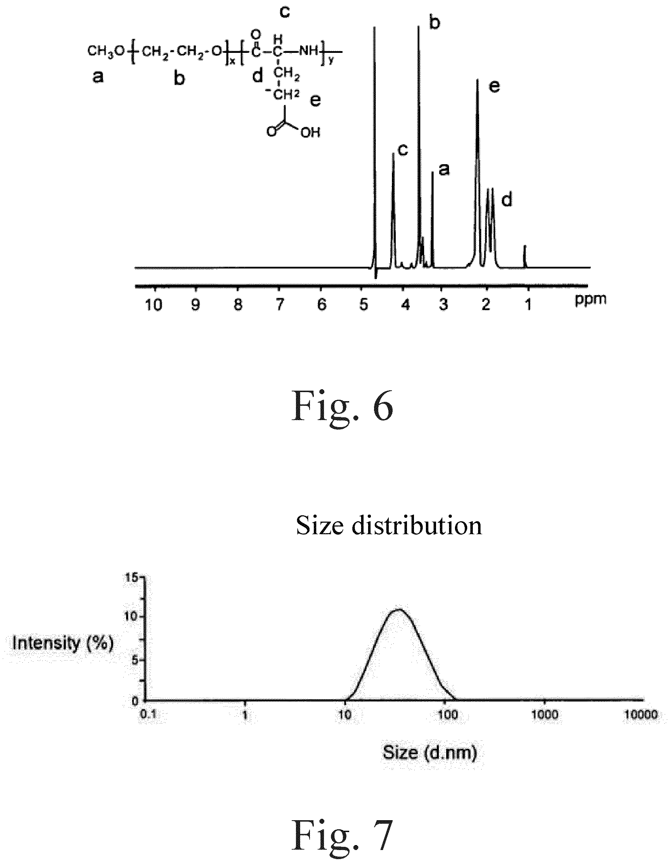

Shown in Figs. 2A and 2B are structures of Amifostine and its derivatives; shown in Fig. 3 is a structure formula of PEG-b-PGA; shown in Fig. 4 is the structure formula of the metal composite prepared by the method illustrated in the aforementioned embodiment; shown in Fig. 5 is gel chromatography analysis of PEG-b-PGA; in the aforementioned embodiment, synthesis of PEG-b-PGA is initiated with PEG as the starting agent, the averaged molecular weight (Mw) is 5700, and number-average molecular weight (Mn) is 4900, polydispersity index (PdI) is 1.16; shown in Fig. 6 is Proton Nuclear Magnetic Resonance (¹H-NMR) spectrum of PEG-b-PGA to demonstrate chemical shifts of the functional groups; shown in Fig. 7 is particle size analysis of the metal composite, and averaged particle size is 31.61 nanometer.

### Embodiment 1

Raw materials included 5 mg of Amifostine, 20 mg of PEG-b-PGA (Mw 5700 Dalton) and 1.25 to 10 mg of FeCl₂, with a weight ratio of 1:4:0.25 to 1:4:2. The prepared raw materials were added in 5 ml buffer solution and undergoes a homogenous agitation procedure at 25°C for 24 hours. The mixture was then placed in a dialysis membrane of Mw 3500 so as to observe self-assembly of the metal composite. Specifically, the buffer solution was 0.05M HEPES(4-(2-hydroxyethyl)-1-piperazineethanesulfonic acid) of pH 7.0.

### Embodiment 2

Raw materials included 5 mg of Amifostine, 20 mg of PEG-b-PGA (Mw 5700 Dalton) and 1.25 to 10 mg of FeCl₂, with a weight ratio of 1:4:0.25 to 1:4:2. The prepared raw materials were added in 5 ml buffer solution and underwent homogenous agitation procedures at 25°C, while the homogenous agitation procedures were performed for 1, 3, 6, 24 hours, respectively. After the homogenous agitation procedure, the prepared metal composites were then placed in a dialysis membrane of Mw 3500 so as to observe their self-assembly. Specifically, the buffer solution was 0.05M HEPES(4-(2-hydroxyethyl)-1-piperazineethanesulfonic acid) of pH 7.0.

The following describes several experimental examples to compare the imaging resolution, contrast enhancement, and residence time of the metal composite provided by this invention and commercial kit used in pericardium and liver imaging.

### Experimental animals and conditions

Table 1 lists detailed information about conditions of the experimental animals used in the following experimental examples and the number of animals that were involved. The experimental animals were 8 to 12 week-old male Sprague-Dawley rats, and they were separated into 2 groups denoted as health and pericarditis, respectively. The metal composites (A01) were administrated to each of the 2 groups in doses of 10 mg/kg and 20 mg/kg, respectively. The control group was administrated of buffer solution, and the comparison group was administrated of commercial bubble contrast agent SonoVue^{®}.

**Table 1**

| Group | strain | Gende r | Age (week) | Weight (g) | control | A01 (10mg/kg ) | A01 (20mg/kg) | SonoVue |
|---|---|---|---|---|---|---|---|---|
| Health | SD | Male | 8 to 11 | 398.8±20.0 | 3 | 3 | 7 | 5 |
| pericarditis | SD | male | 8 to 12 | 475.7±14.4 | 5 | 3 | 3 | 2 |

Table 2 further lists the parameter settings for ultrasound scanning of the rat heart in the following experimental examples. In the experimental examples, a linear ultrasonic scanner was utilized with a frequency of 7.1 MHz, a scan depth of 2.4 cm, a Gain setting of 60, an image enhancement setting of 3, and a scan area of 36 cm². Images were captured by scanning at 0, 10, 30, 60 and 120 minutes post administration of the metal composites (A01).

**Table 2**

| Frequency (MHz) | Scan depth (cm) | Gain | Image enhancement | Scan area (cm²) |
|---|---|---|---|---|
| 7.1 | 2.4 | 60 | 3 | 6 * 6 |

### Experimental example 1: cardiac ultrasound images at various time point post metal composite A01 administration

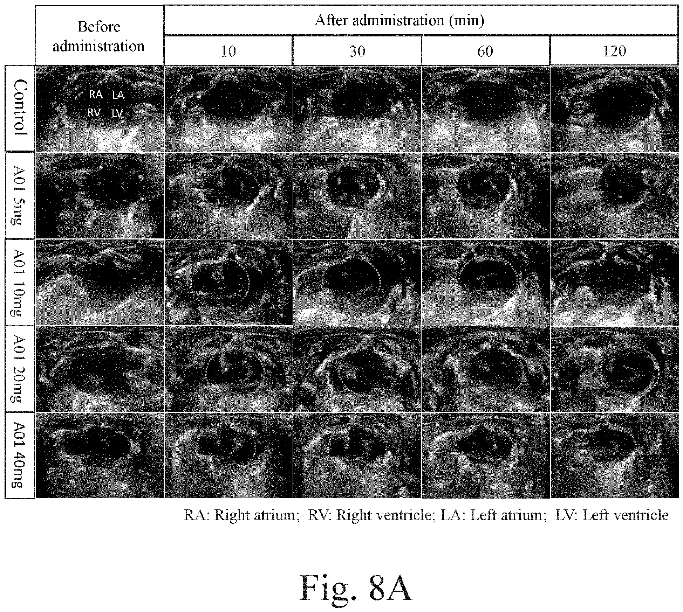

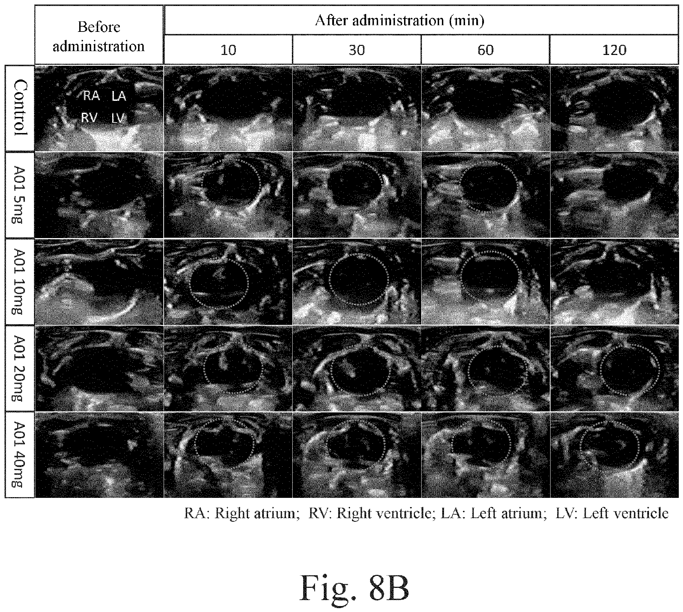

Figs. 8A to 8B demonstrate cardiac ultrasound images of rat hearts at systolic and diastolic phases, respectively. The cardiac ultrasound images were performed before and after administration of the metal composite prepared in embodiment 1. In doses of 5 or 10 mg/kg, the ultrasonic contrast of heart could last for 60 minutes, while in doses of 20 or 40 mg/kg, the ultrasonic contrast of heart could last even longer for 120 minutes.

**Table 3**

| Image brightness | After administration (min) | | | | | % of Peak |
|---|---|---|---|---|---|---|
| | 0 | 10 | 30 | 60 | 120 | |
| control | 0 | -1 | 0.7 | -1.3 | -0.6 | - |
| 5 mg/kg | 0 | 8.9 | 13.5 | 8.6 | 2.8 | 21 |
| 10 mg/kg | 0 | 11.2 | 11.5 | 6.7 | 3.8 | 33 |
| 20 mg/kg | 0 | 12.3 | 11.4 | 7.3 | 7.5 | 61 |
| 40 mg/kg | 0 | 12.1 | 15.7 | 11.4 | 17.4 | 111 |

Please refer to Table 3, at a dose of 10 or 20 mg/kg, brightness of ultrasonic contrast peaked 10 minutes after administration, and decreased to 33 to 61 % of the peak value 120 minutes after administration. At a dose of 5 or 40 mg/kg, brightness reached a first peak 30 minutes after administration, and at a dose of 5 mg/kg, the brightness decreased to 21 % of the peak value 120 minutes after administration, while at a dose of 40 mg/kg, the brightness was elevated to 111% of the peak value 120 minutes after administration.

On the other hand, please refer to Table 4, at a dose of 10 or 20 mg/kg, intensity of the ultrasonic contrast peaked 10 minutes after administration, and decreased to 33 to 60% of the peak value 120 minutes after administration. At a dose of 5 or 40 mg/kg, intensity reached a first peak 30 minutes after administration, and at a dose of 5 mg/kg, the intensity decreased to 21 % of the peak value 120 minutes after administration, while at a dose of 40 mg/kg, the intensity was elevated to 112% of the peak value 120 minutes after administration.

**Table 4**

| intensity | After administration(min) | | | | | % of Peak |
|---|---|---|---|---|---|---|
| | 0 | 10 | 30 | 60 | 120 | |
| control | 0 | -17247.7 | 13296 | -23145.8 | -10951.7 | - |
| 5 mg/kg | 0 | 158268 | 239228 | 154713 | 49389 | 21 |
| 10 mg/kg | 0 | 198304 | 205184 | 161911 | 67667 | 33 |
| 20 mg/kg | 0 | 220072 | 202870 | 129503 | 132215 | 60 |
| 40 mg/kg | 0 | 214959 | 276160 | 201251 | 308780 | 112 |

### Experimental example 2: comparison to commercial contrast SonoVue in continuous cardiac ultrasound images

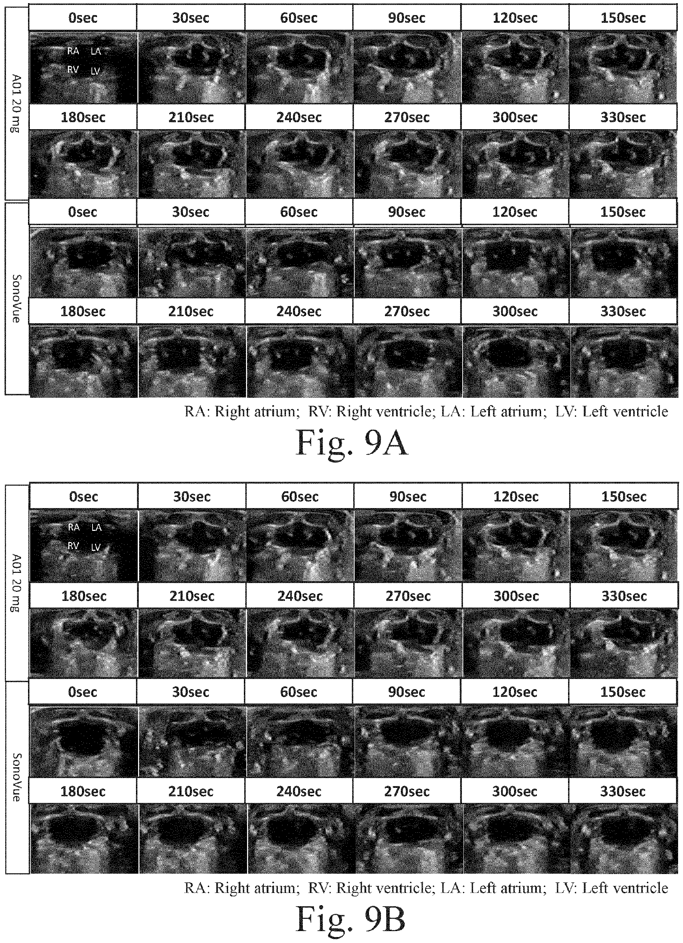

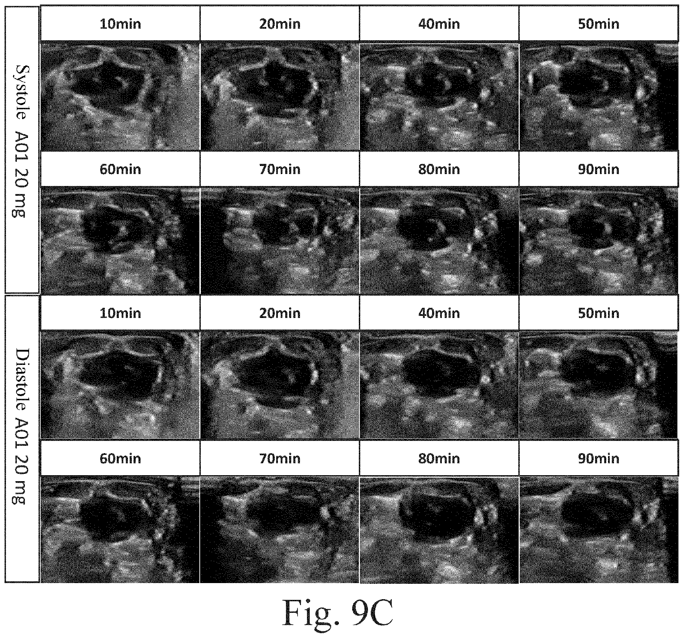

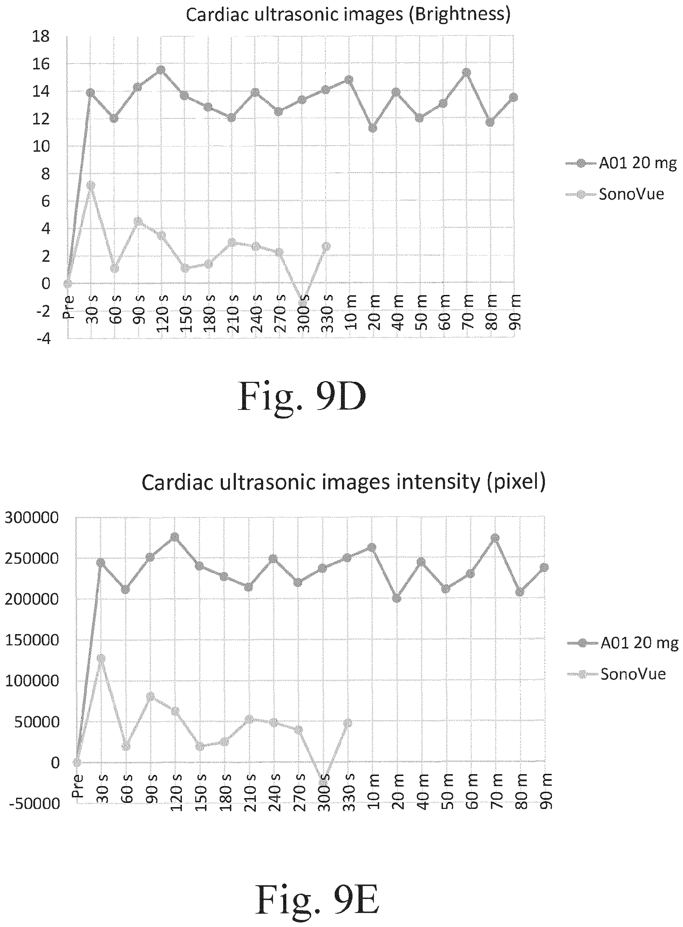

Figs. 9A to 9B demonstrate cardiac ultrasound images of rat hearts at systolic and diastolic phases, respectively. In this experimental example, durations of cardiac ultrasound images were compared in parallel after administration of the metal composite A01 prepared in embodiment 1 and the commercial contrast SonoVue, respectively. The cardiac ultrasound images were performed before and after administration of the metal composite prepared in embodiment 1. At a dose of 20 mg/kg, with ultrasonic contrast conducted every 30 seconds, ultrasound image of cardiac valves remained clear 330 seconds after A01 administration. In contrast, ultrasound image of cardia valves in the group administrated of SonoVue became unobservable gradually 270 seconds after administration. On the other hand, Fig. 9C further demonstrates that cardiac ultrasound images remained effective imaged, in comparison with SonoVue, 90 minutes after A01 administration at a dose of 20 mg/kg.

Figs. 9D to 9E illustrates brightness and intensity analysis of the cardiac ultrasound images to indicate trends of brightness and intensity at different time points after metal composite A01 and SonoVue administration. Table 5 lists analysis of cardiac ultrasound images brightness and intensity data obtained by using image analytic software ImageJ. On condition that the average value of SonoVue is 1.00, averaged brightness and intensity at each time point within 330 after A01 administration were 5.34 and 5.29 times of SonoVue, respectively. This indicates that A01 significantly outperforms SonoVue in terms of both cardiac ultrasound images brightness and intensity.

**Table 5**

| Rat cardiac ultrasound images | | |
|---|---|---|
| | Ave. brightness | Ave. intensity |
| A01 | 5.34 | 5.29 |
| SonoVue | 1.00 | 1.00 |

### Experimental example 3: comparison to commercial contrast SonoVue in continuous cardiac ultrasound images of rat pericardium

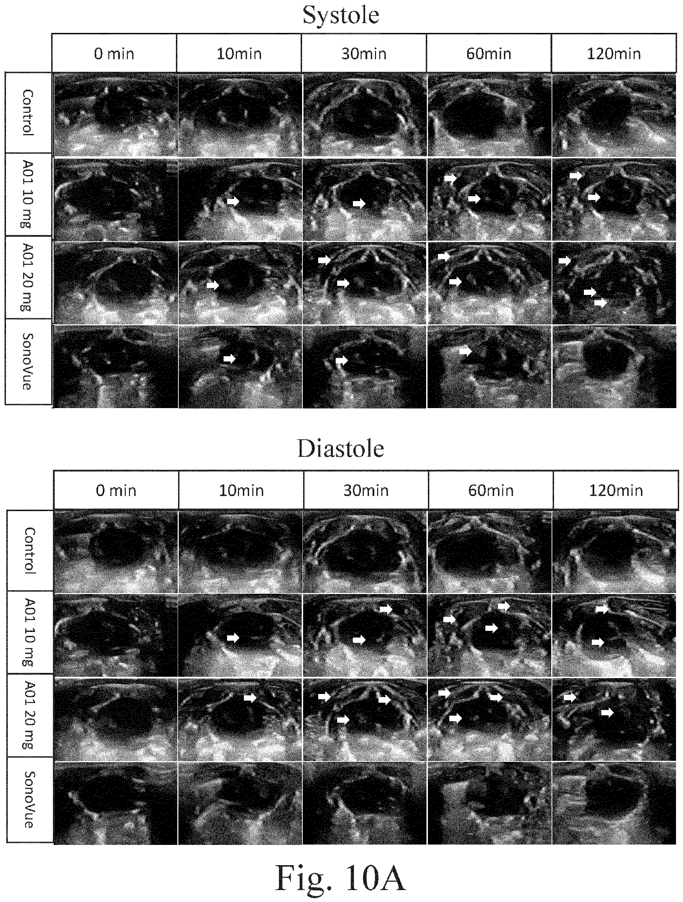

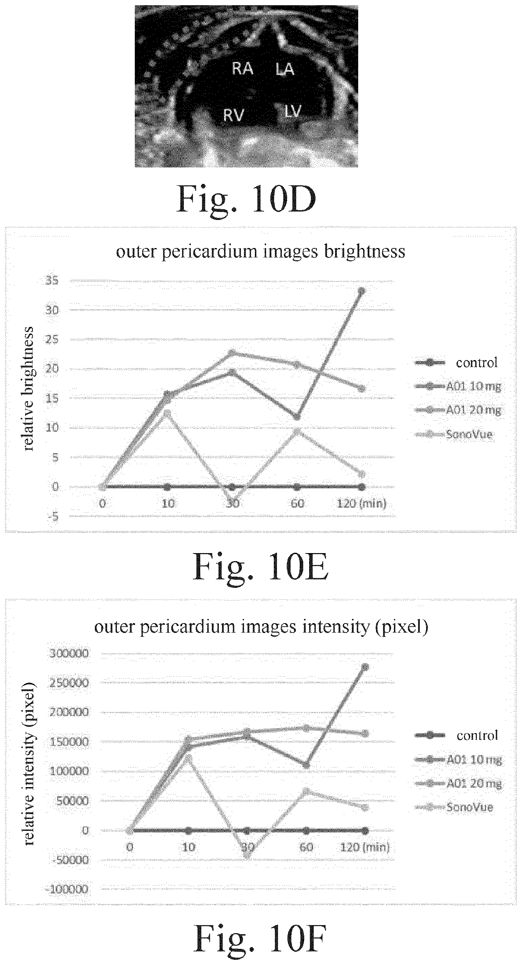

Acute pericarditis was induced by needle puncture through the rat heart, and cardiac ultrasound images was conducted to observe the outer pericardium images with metal composite A01. With reference to Fig. 10A, which illustrates cardiac ultrasound images of rat hearts at systolic and diastolic phases, respectively. In this experimental example, the imaging effect of cardiac ultrasound images was compared in parallel after 10mg/kg or 20mg/kg administration of the metal composite A01 and the commercial contrast SonoVue, respectively. as indicated by white arrow, the heart valves of both groups could be clearly identified 10 minutes after administration. At 30 minutes after administration, contour of the outer pericardium of the group at dose of 20mg/kg remained clear (white arrow on upper left corner). At 60 minutes after administration, contour of the outer pericardium and heart valves of both groups at low dose and high dose could be clearly observed, and the imaging remained clear 120 minutes after administration. The commercial SonoVue, however, produced unclear contour of the heart valves and outer pericardium 60 minutes after administration.

**Table 6**

| Heart valve images of rat with acute pericarditis | | |
|---|---|---|
| | Ave. Brightness | Ave. intensity |
| Control | 1.00 | 1.00 |
| 10 mg/kg | 6.42 | 6.32 |
| 20 mg/kg | 13.58 | 13.38 |
| SonoVue | 6.10 | 6.00 |

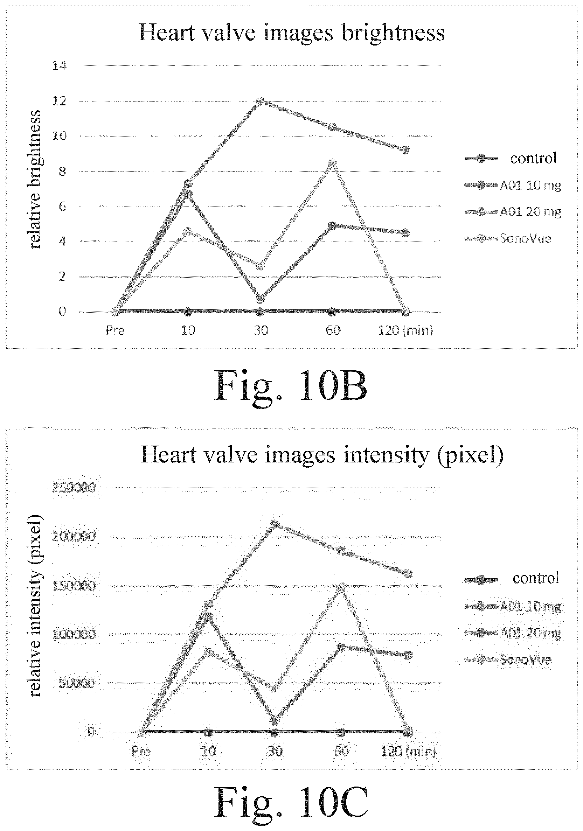

Please refer to Table 6 and Figs. 10B to 10C, which illustrates brightness and intensity of acute pericarditis heart valve images that were obtained by ImageJ analysis. The brightness values and intensity values were obtained at different time points after A01 administration and were averaged. On condition that averaged value of control group was 1.00, the relative averaged values of brightness and intensity were 6.42, 6.32 (10 mg/kg), and 13.58, 13.38 (20 mg/kg). This indicates that A01 can significantly enhances both brightness and intensity of heart valve images in acute pericarditis rats after both low and high dose administrations. Regarding SonoVue, with reference to Figs. 10B to 10C, SonoVue produced fair brightness and intensity of image 60 minutes after administration, but could not maintain the imaging effect at 120 minutes after administration. At time points beyond 120 minutes, the brightness and intensity decreased to an unobservable level.

Please refer to Figs. 10C to 10E and Table 7, which illustrates brightness and intensity of outer pericardium images that were obtained by ImageJ analysis, and the data was obtained from outer periphery of the pericardium as selected by the red dot line in Fig. 10D. The brightness values and intensity values were obtained at different time points after A01 administration and were averaged. On condition that averaged value of control group was 1.00, the relative averaged values of brightness and intensity were 15.36, 15.13 (10 mg/kg), and 14.36, 14.50 (20 mg/kg). This indicates that A01 can significantly enhances both brightness and intensity of heart valve images in acute pericarditis rats after both low and high dose administrations, and such enhancement was 2 to 3 times greater than that of SonoVue in terms of both brightness and intensity. Similarly, with reference to Figs. 10E to 10F, SonoVue produced fair brightness and intensity of image 60 minutes after administration, but could not maintain the imaging effect at 120 minutes after administration. At time points beyond 120 minutes, the brightness and intensity decreased to an unobservable level.

**Table 7**

| Rat outer pericardium images | | |
|---|---|---|
| | Ave. Brightness | Ave. intensity |
| Control | 1.00 | 1.00 |
| 10mg/kg | 15.36 | 15.13 |
| 20mg/kg | 14.36 | 14.50 |
| SonoVue | 4.86 | 4.80 |

### Experimental example 4: comparison to commercial contrast SonoVue in continuous rat hepatic ultrasound images

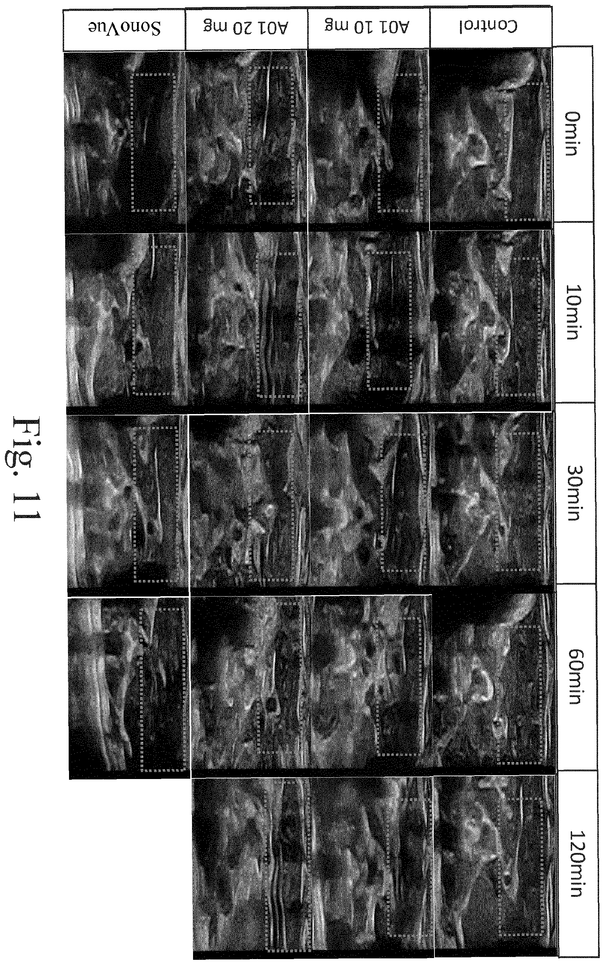

With reference to Fig. 11, which illustrates images of rat hepatic ultrasound images. In this experimental example, durations of the hepatic ultrasonic contrast effect were compared in parallel after 10mg/kg or 20mg/kg administration of the metal composite A01 and the commercial contrast SonoVue, respectively. At both doses of 10 mg/kg and 20 mg/kg, ultrasound images could be clearly observed at 10, 30, 60 and 120 minutes after A01 administration, while hepatic ultrasound images were unidentifiable 60 minutes after SonoVue administration.

**Table 8**

| Rat hepatic ultrasound images | | |
|---|---|---|
| | Ave. Brightness | Ave. intensity |
| 10mg/kg | 2.02 | 1.84 |
| 20mg/kg | 1.64 | 1.51 |
| SonoVue | 1.00 | 1.00 |

On the other hand, Table 8 lists brightness and intensity analysis of hepatic ultrasound images so as to demonstrate brightness and intensity data analysis at each time point after A01 and SonoVue administration, respectively. On condition that averaged value of SonoVue was 1.00, averaged brightness and intensity 120 minutes after A01 administration were 2.02 and 1.84 times (10mg/kg), and 1.64 and 1.51 times (20mg/kg) greater than that of SonoVue. Clearly, hepatic ultrasonic contrast of A01 outperforms SonoVue in terms of both brightness and intensity.

The known metal composite forms a microbubble structure that tends to collapse under ultrasound. While the known metal composite can act as a drug carrier, it is not suitable for long-term observation of organ changes using ultrasonic contrast. The microbubble structure of the known metal composite is not expected to sustain its imaging effects for an extended period after administration.

The metal composite provided in this invention is structurally designed to be a nano-metal micelle. Based on coordinate-covalent bond, the nano-metal micelle possesses a chelator-like structure by using the metal ion as Lewis acid to chelate contrast ligand having Lewis base functional group and shell ligand having polymer. The chelator-like structure lengthens half-life of the metal composite in organ tissues. The metal composite in this invention is prepared from highly biocompatible materials that can be metabolized by human body and toxic-free.

Moreover, metal ion itself tends to precipitate. To improve dispersity of the metal composite, PEG having excellent dispersibility is used as dispersing group of the shell ligand. Meanwhile, with PGA modification to form the chelating group, a coordinate-covalent bond can be formed between the shell ligand and the metal ion. The nano-metal micelle can therefore be homogenously dispersed in a fluid, and precipitation caused by the metal ion could be prevented, which extends shelf life and ensures structural integrity of the metal composite in circulation, and ultrasonic contrast effects can sustain for a longer period.

In another aspect, the high dispersibility of the shell ligand renders preparation of the metal composite in this invention easy. Preparation of the metal composite can be accomplished by self-assembly reaction under through the chelating effect of the ligands under homogenous agitation procedures on at constant temperature, which benefits massive production.

In one another aspect, the metal composite in this invention is also verified to be applicable for multiple types of organ tissues, and outperforms commercial microbubble metal composite to demonstrate higher brightness and intensity in cardiac ultrasound images and hepatic ultrasound images. The metal composite in this invention demonstrate significantly better contrast enhancement than that of commercial microbubble metal composite, and imaging duration after administration can sustain multiple times and even dozens of times longer. In the near future, portable ultrasonic contrast device will be widely available, and operation of family-used imaging will need longer time for less seasoned users. With the metal composite in this invention, the less seasoned users are free from limit of short effective period of conventional imaging contrasts, and they are benefited by long effective imaging duration achieved by this invention. These users can avoid repetitive administration of imaging contrasts within a short period, and ultrasonic contrast becomes cost effective and easy to promote.

## Claims

1. A metal composite for ultrasonic contrast, comprising:
a contrast core comprising a metal ion; and
a shell ligand comprising a polymer having a chelating group, wherein the chelating group is a Lewis base and forms a coordinate-covalent bond with the metal ion so as to form a stable metal nano-structure.

2. The metal composite according to claim 1, wherein the contrast core comprises an contrast ligand, and the contrast ligand has a Lewis base functional group, and forms coordinate-covalent bond with the metal ion via the Lewis base functional group, wherein the Lewis base functional group of the contrast ligand comprises any one of a primary carbon having a π bond, a secondary carbon, a group 16 element, a primary amine, a secondary amine, a tertiary amine or a combination of two or more thereof.

3. The metal composite according to claim 2, wherein the contrast ligand comprises a chemical structure as a constitutional formula (1): 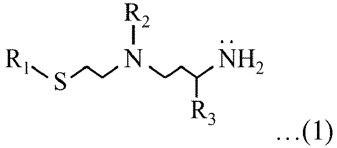 wherein R₁ is selected from a group consisting of hydrogen, hydroxyl group, carboxylic group, alcoholic group, ketone group, furans, amine, anilines, pyrroles, esters, amides, imines, pyridines, pyrimidins, imidazoles, pyrazols, phosphonic acids, sulfide and disulfide; R₂ is selected from a group consisting of hydrogen _{'} hydroxyl group, carboxylic group, alcoholic group, ketones, furans, amines, anilines, pyrroles, thiols, esters, amides, imines, pyridines, pyrimidines, imidazoles, pyrazols, sufonamides and phosphonic acid;R₃ is selected from a group consisting of hydrogen, hydroxyl group and carboxylic group.

4. The metal composite according to claim 2, wherein the contrast ligand is selected from a group consisting of Amifostine, Amifostine thiol (WR-1065), Amifostine disulfide (WR-33278), Doxorubicin, Docetaxel, Epirubicin, Amphotericin, Ciprofloxacin or a combination of two or more thereof.

5. The metal composite according to any one of claims 1 to 3, wherein the metal ion is selected from a group consisting of Fe, Cu, Ni, In, Ca, Co, Cr, Gd, Al, Sn, Zn, W, Sc, Ti, Mn, V, Mg, Be, La, Au, Ag, Cd, Hg, Pd, Re, Tc, Cs, Ra, Ir, Ga and a combination of two or more thereof.

6. The metal composite according to any one of claims 1 to 3, wherein the chelating group is selected from a group consisting of poly(aspartic acid), poly(glutamic acid), polylysine, poly(acrylic acid), polyethyleneimine, poly(methacrylic acid), hyaluronic acid, poly(N-isopropyl acrylamide), poly(lactic acid), poly(butylenesuccinate) and a combination of two or more thereof.

7. The metal composite according to claim 6, wherein the polymer further comprises a dispersing group selected from a group consisting of polyethylene glycol), chitosan, collagen, poly(N-isopropyl acrylamide), amylose, cellulose, poly(hydroxybutyrate), poly(caprolactone), carboxymethylcellulose, dextran, cyclodextrin and a combination of two or more thereof.

8. A use of a metal composite for preparation of an organism imaging composition, comprising administrating an effective dose of the organism imaging composition to a vasculature of a location in need, wherein the organism imaging composition comprises the metal composite according to claim 1.

9. The use according to claim 8, wherein the contrast core comprises an contrast ligand, and the contrast ligand has a Lewis base functional group, and forms coordinate-covalent bond with the metal ion via the Lewis base functional group, wherein the Lewis base functional group of the contrast ligand comprises any one of a primary carbon having a π bond, a secondary carbon, a group 16 element, a primary amine, a secondary amine, a tertiary amine or a combination of two or more thereof.

10. The use according to claim 8 or 9, wherein the location in need comprises human organ tissue including heart, liver, spleen, lung, kidney, pancreas, esophagus, stomach, intestine, colon, lymphatic system or reproductive system.
